(19)

Europäisches Patentamt
European Patent Office
Office européen des brevets

(11)  **EP 4 248 202 B1**

(12)  **EUROPEAN PATENT SPECIFICATION**

(45) Date of publication and mention of the grant of the patent:
**08.04.2026  Bulletin 2026/15**

(21) Application number: **21901182.2**

(22) Date of filing: **02.12.2021**

(51) International Patent Classification (IPC):
**G01N 27/327** (2006.01)  **G01N 27/02** (2006.01)

(52) Cooperative Patent Classification (CPC):
**G01N 27/028; G01N 27/416; G01N 27/49; G01N 33/48707**

(86) International application number:
**PCT/TR2021/051337**

(87) International publication number:
**WO 2022/119546 (09.06.2022 Gazette 2022/23)**

(54) **A MINIATURIZED COMPACT ELECTROCHEMICAL ANALYZER**

KOMPAKTER ELEKTROCHEMISCHER ANALYSATOR MIT MINIATURISIERTER GRÖSSE

ANALYSEUR ÉLECTROCHIMIQUE COMPACT MINIATURISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **03.12.2020  TR 202019655**
**19.11.2021  TR 202118093**

(43) Date of publication of application:
**27.09.2023  Bulletin 2023/39**

(73) Proprietor: **Ege Universitesi**
**35100 Izmir (TR)**

(72) Inventors:
• **DEMIRHAN, Alper**
**35100 Bornova/ zmir (TR)**
• **ERDEM GÜRSAN, Kadriye Arzum**
**35100 Bornova/ zmir (TR)**

(74) Representative: **Potter Clarkson**
**Chapel Quarter**
**Mount Street**
**Nottingham NG1 6HQ (GB)**

(56) References cited:
CN-A- 101 871 908      US-A1- 2010 204 936
US-A1- 2012 298 530    US-A1- 2018 224 394

• CARMINATI MARCO ET AL: "Attofarad resolution potentiostat for electrochemical measurements on nanoscale biomolecular interfacial systems", REVIEW OF SCIENTIFIC INSTRUMENTS, AMERICAN INSTITUTE OF PHYSICS, 2 HUNTINGTON QUADRANGLE, MELVILLE, NY 11747, vol. 80, no. 12, 4 December 2009 (2009-12-04), pages 124701 - 124701, XP012128050, ISSN: 0034-6748, DOI: 10.1063/ 1.3245343
• MATSUBARA YASUO: "A Small yet Complete Framework for a Potentiostat, Galvanostat, and Electrochemical Impedance Spectrometer", JOURNAL OF CHEMICAL EDUCATION, vol. 98, no. 10, 12 October 2021 (2021-10-12), US, pages 3362 - 3370, XP093141042, ISSN: 0021-9584, DOI: 10.1021/acs.jchemed.1c00228

## Description

### Technical Field Related to the Invention

The invention relates to an electrochemical analyzer, in which both the electrochemical impedance spectroscopy and the potentiostat circuits are miniaturized on a single board and made compact.

### State of the Art of the Invention (Prior Art)

US2018/224394A1 discloses systems, methods, and the like, for an electrochemical sensing platform for point-of-care diagnostic applications. The article by Carminati, M., Ferrari, G. Samplietro, M., titled "Attofarad Resolution Potentiostat for Electrochemical Measurements on Nanoscale Biomolecular Interfacial Systems", in Review of Scientific Instruments, vol. 80, 124701, 2009 discloses an instrument that enables electrochemical measurements (cyclic voltammetry, impedance tracking, and impedance spectroscopy) on submicrometric samples. The article by Matsubara, Y., titled "A Small yet Complete Framework for a Potentiostat, Galvanostat, and Electrochemical Impedance Spectrometer", in J. Chem. Educ., 2021, 98, 3362-3370 describes a general-purpose, scalable, handheld (10 cm2), yet affordable hardware-software framework for a potentiostat, galvanostat, and electrochemical impedance spectrometer with high capabilities: (1) cyclic voltammetry, square-wave voltammetry, and (potential-step) chronoamperometry as potentiostatic measurements conjugated with iR compensation and rotating-disk electrode apparatus in a wide range of current and voltage (pA to 0.1 A and $\pm$12 V); (2) chronopotentiometry (with an arbitrary current) as a galvanostatic measurement (e.g., Karl Fischer titration); and (3) electrochemical impedance spectroscopy with a bandwidth of 0.1-1MHz.

There is currently no structure to calibrate the signal distorting effects arising from the circuit elements in the devices used. Therefore, they have to use more expensive and high-performance circuit elements. Both expensive and energy consuming structures such as FPGA are used for signal processing.

### Brief Description and Objects of the Invention

The said invention is described in claim 1 and relates to a miniaturized compact electrochemical analyzer device in order to bring new advantages to the field related to eliminating the above-mentioned disadvantages.

The object of the present invention is to develop a handheld electrochemical analyzer, comprising both the electrochemical impedance spectroscopy and the potentiostat circuits in a compact state. The available electrochemical analyzers are generally large in size and costly devices that consume high energy in this context. Impedance spectroscopy and potentiostat circuits are integrated on a single board in the invention considering these disadvantages. Thus, the performance of the device has increased compared to the existing devices, the dimensions of the device have become smaller compared to the existing devices, the cost has decreased and the integration of the device into the innovative biosensors in particular has become easier.

It can also be used for handheld applications as it is both miniaturized and compact and can perform all operations without the need for an external computer such as desktop.

Electrochemical analysis of electroactive species can be carried out using alternating voltammetry, differential pulse voltammetry, square wave voltammetry, linear sweep voltammetry, normal pulse voltammetry, chronoamperometry, amperometry and electrochemical impedance spectroscopy (EIS), etc. methods with the miniaturized compact electrochemical analyzer developed within the scope of the invention.

It is possible to apply to the electrochemical analysis of target analytes (medicine, environmental pollutant species, toxins, food additives, etc.) as well as many biomolecules (DNA, RNA, protein, etc.) with the developed electrochemical analyzer.

This electrochemical analyzer, which has been developed for the electrochemical analysis of target analytes in the medical, environmental, defense and food sector, can also be used in the development of (bio) sensors that allow analysis at the bedside/in the field.

### Definitions of Figures Describing the Invention

The descriptions of the figures are listed below for a better understanding of the invention.

Figure 1. It shows the block diagram of the device.
Figure 2. It shows the circuit diagram used at the TIA (Transimpedance Amplifier) output to remove the background current.
Figure 3. It shows the spectrum of impedance magnitude up to 100 kHz of resistance values between 3.41 kΩ and 46.95 kΩ.
Figure 4. It shows the phase spectrum of Figure 3.

### Element Listing

The numbers of the elements and the elements in the figures are listed below for a better understanding of the invention.

| 1 | Direct digital synthesizer (DDS) |
| 2 | Multiplying digital-to-analog converter |
| 3 | Feedback amplifier |
| 4 | Analog switch |
| 5 | Counter electrode (CE) |
| 6 | Reference electrode (RE) |
| 7 | Working electrode (WE) |
| 8 | Transimpedance amplifier (TIA) |

9 Background removal circuit (BRC)
10 Low-speed analog-to-digital converter (LSADC)
11 High-speed analog-to-digital converter (HSADC)
12 USB
13 Microcontroller
14 Analog signal output from the internal digital-to-analog converter (DAC) of the microcontroller
15 Operational amplifier
16 Operational amplifier output connected as input to analog-to-digital converters
17 R2 gain resistance
18 R1 gain resistance
19 TIA output signal

## Detailed Description of the Invention

The innovation subject to the invention is only for a better understanding of the subject and is explained in detail with examples in a way that does not have any limiting effect in this detailed description.

The said invention relates to an electrochemical analyzer device.

Integration of EIS and potentiostat circuits: Both the EIS and the potentiostat circuit were integrated into a small and inexpensive structure by using the features of the mixed signal processor used. The solution proposal developed and presented has basically 3 important differences according to the situation:

1. Automatic calibration circuit: A circuit capable of instant calibration has been added to the circuit automatically during the experiment in order to eliminate the effects such as voltage difference and phase difference arising from the non-ideality of the circuit elements. Thus, high-performance results were obtained with low-cost components.

2. Signal generator circuit: Direct digital synthesizer chip was used for signal generation, thus generating high resolution and high frequency AC signal. Multiplying digital-to-analog converter circuit was used to change the amplitude of the signal. Thus, an advantage over structures that are normally performed with high-speed digital-to-analog converters and processors, and thus consume more energy and cost more.

3. Integration of EIS and potentiostat circuits: Both the EIS and the potentiostat circuit were integrated into a small and inexpensive structure by using the features of the mixed signal processor used.

- It is a compact electrochemical analyzer created by miniaturizing both EIS and potentiostat circuits on a single board.

- A calibration circuit has been added for the EIS, so that a simple but high-performance system has been developed by calibrating separately at the end of each experiment.

- Using a microcontroller (MC) containing most peripherals, there is no need to externally use analog-to-digital converter (ADC), DAC, and USB (Universal Serial Bus) circuits for the potentiostat circuit. These peripherals can be calibrated to give similar performance results with other devices.

- Wider bandwidth measurements have been provided by using digital signal processing techniques. For example, the excitation and measurement signal frequency is limited only by the speed of the ADC and DAC since no analog filter is used to filter the 50/60 Hz mains line.

- The analysis of the target analytes can be carried out by using electrochemical techniques such as voltametric, impedimetric, amperometric, etc. with the electrochemical analyzer developed within the scope of the invention.

- It is possible to apply to the electrochemical analysis of target analytes (medicine, environmental pollutant species, toxins, food additives, etc.) as well as many biomolecules (DNA, RNA, protein, etc.) with the developed electrochemical analyzer.

- A circuit that will remove the background current electronically has been realized in measurements made by applying a pulse such as differential pulse voltammetry thanks to the background removal circuit.

- An analog switch has been added to short the reference electrode and the working electrode, enabling the device to be used for two-electrode systems.

The said invention is an electrochemical analyzer device, characterized in that it comprises the following elements;

- At least one microcontroller (13) containing internally low-speed (at least 16-bit) analog-digital converter (ADC) and digital-analog converter (DAC), providing communication with other elements in the circuit, communicating with other computers via USB (12),
- At least one control circuit in which potentiostat ADC with potentiostatic signal generator, USB (12) and the processor are collected in a single integrated circuit to reduce the dimensions of the device and reduce the cost,
- At least one voltage amplifier circuit for potentiostatic measurements in order to make measurements in the range of $\pm 10$ V,

- At least one transimpedance amplifier (TIA) (8) circuit, which allows the current gain to be determined with a digital potentiometer,
- At least one multiplying DAC circuit that determines the signal amplitude and scales the sinusoidal signal depending on the feedback from the reference electrode (RE) (6) for impedance analysis,
- At least one automatic calibration circuit consisting of a digital potentiometer connected in parallel with the Transimpedance Amplifier (TIA) (8) circuit, which provides instant calibration to calibrate errors caused by non-ideal situations such as leakage current, voltage differences and temperature,
- At least one working electrode (WE) (7), which provides both impedance analyzer excitation signal and potentiostat excitation signal,
- At least one reference voltage generator circuit connected to the power pins of these circuits to supply analog circuits and working electrode,
- Background removal circuit (BRC) (9) for removing background signals,
- Low-speed analog-to-digital converter (LSADC) (10) circuit with a resolution of 6-bit to 32-bit and a sampling rate of less than one million per second to read low-frequency signals,
- High-speed analog-to-digital converter (HSADC) (11) circuit with a resolution of 6-bit to 32-bit and a sampling rate of more than one million per second to read electrochemical impedance spectroscopy signals (especially high-frequency signals at 1 kHz),
- USB (12) circuit that can communicate with devices such as computers, tablets, smartphones with the internal USB (12) controller of the microcontroller (13) and receives power from these devices,
- Operational amplifier (15) in non-inverting feedback structure, which is used only to amplify Faradaic currents in potentiostat protocols,
- Analog switch (4) circuit, which shorts the reference electrode and the working electrode and enables them to be used in 2-electrode systems,
- R1 gain resistance (18) and R2 gain resistance (17), which provide voltage from the internal digital-to-analog converter of the microcontroller (13) to the positive pin of the amplifier in the background removal circuit (BRC) (9) and increase the difference $1 + \frac{R2}{R1}$ between this voltage and the output of the Transimpedance amplifier (TIA) (8).

Adjusting the voltage applied to the said electrodes by multiplying digital-to-analog converter

- The use of the potentiostat and impedance analyzer in the analysis of numerous and different target analytes (nucleic acid, protein, amino acid, drug, toxin, environmental pollutant, bio-threat element species, etc.) in the health, environment, defense, food fields.

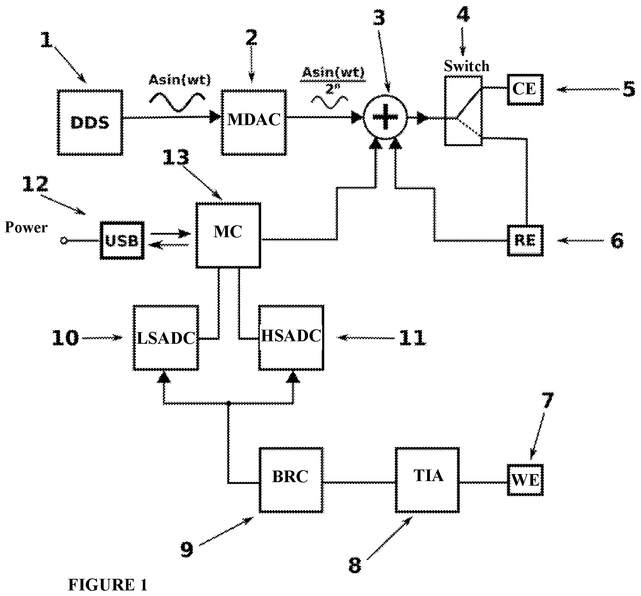

Figure 1 shows the general diagram of the device. The direct digital synthesizer (1) (DDS) generates a sine signal indicated by Asin(wt), the value A of which can be between 0.1 mV and 1 V. The frequency of the generated signal may be from 0.001 Hz to 10 MHz. This fixed amplitude signal is applied as a reference signal to the multiplying digital-to-analog converter (2) (MDAC). The resolution of the MDAC can be between 6-bit and 24-bit. The MDAC divides this reference signal into $2^n$ parts, n being the number of bits, to form an AC signal to be applied to the electrodes. Thus, the voltage to be applied to the electrodes is adjusted in software to be of minimum $\frac{Asin\,(wt)}{2^n}$ size.

The DC voltage to be applied to the electrodes using the internal DAC of the microcontroller (13) (MC) is added to the AC signal (3). The total result is applied to the counter electrode (CE) (5) with the feedback from the reference electrode (RE) (6). The analog switch (4) is a programmable analog switch (4) that short-circuits RE and CE. The current from the sensor flows through the working electrode (7) (WE) and is converted into voltage through the transimpedance amplifier (8) (TIA). The background removal circuit (9) (BRC) increases the voltage at the TIA output by proportioning it to a voltage proportional to the background voltage to remove non-faradaic currents. Low-speed analog-to-digital converter (10) (LSADC) is an integrated circuit with a sampling rate of less than 1 million per second and a resolution of 6-bit to 32-bit values. High-speed analog-to-digital converter (11) (HSADC) is an integrated circuit with a sampling rate of more than 1 million per second and a resolution between 6-bit and 32-bit values. The microcontroller (13) (MC) communicates with all other elements and communicates with other computers via USB. It includes internal USB drive, LSADC, and DAC..

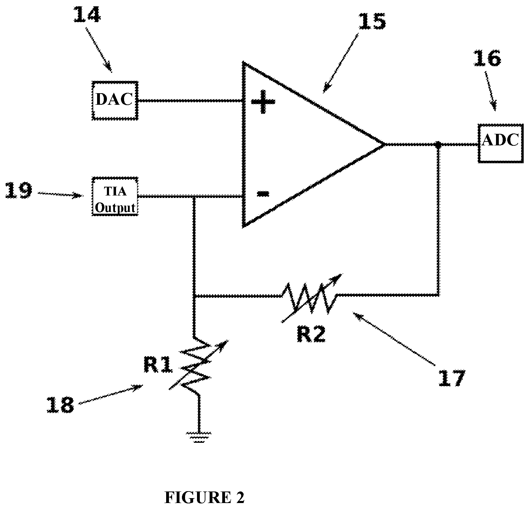

BRC is shown in Figure 2. The Transimpedance amplifier (TIA) (8) signal and the TIA output signal (19) from the TIA are amplified again at a rate determined by R1 gain resistance (18) and R2 gain resistances (17), being the signal base signal provided from the internal DAC of the MC. This is done with an operational amplifier (15). The output of the operational amplifier (15) is connected to the analog-digital converters (ADC) as input.

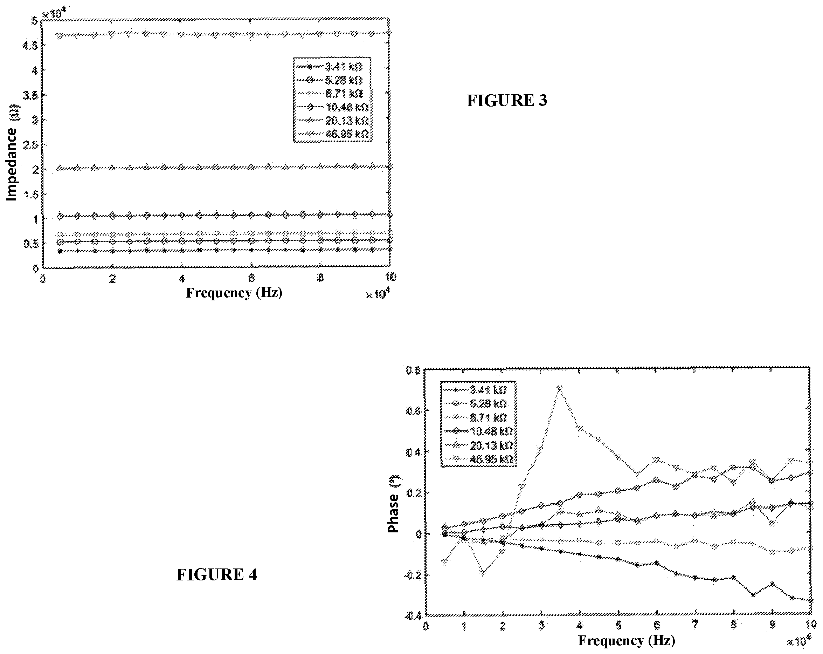

Figure 3 shows sample measurements made with 46.95 kΩ, 20.13 kΩ, 10.48 kΩ, 6.71 kΩ, 5.28 kΩ, and 3.41 kΩ. The figure shows the amplitude response of the frequency spectrum of resistors.

Figure 4 shows the phase spectrum of the measurement in which the amplitude response is given in figure 3.

A method of operation of the device, characterized in that it comprises the following steps;

- The direct digital synthesizer (1) (DDS) generating a fixed amplitude sine signal,
- Applying this fixed amplitude signal as a reference signal to the multiplying digital-to-analog converter (2) (MDAC),

- Creating an AC signal with an amplitude ranging from 0.1 mV to 1 V by scaling the reference signal to the multiplying digital-to-analog converter (2) (MDAC) as 6-bit or 24-bit,
- Adding the DC voltage to be applied to the electrodes using the internal digital-analogue converter (DAC) of the microcontroller (13) (MC) with the AC signal,
- Applying the total result to the counter electrode (CE) (5) to keep the feedback voltage received in the reference electrode (RE) (6) at the desired value,
- The current from the sensor flowing through the working electrode (WE) (7) and being converted into voltage through the transimpedance amplifier (8) (TIA),
- The background removal circuit (BRC) (9) increasing the voltage at the TIA output by proportioning it to a voltage proportional to the background voltage to remove non-faradaic currents,
- Re-amplification of the signal (19) from the TIA with an operational amplifier (15) at a rate determined by R1 gain resistance (18) and R2 gain resistance (17), being the signal (13) base signal provided from the internal DAC of the MC
- Connecting the output of the operational amplifier to the analog-digital converters (ADC) as input,
- Transmitting the result of the analysis to electronic environments such as computers, tablets, etc.,
- Converting the result to a measurable numerical value.

The method can be adapted to multiple electrode systems with common reference and common counter electrodes as applied to single electrodes in the preferred embodiment of the invention.

**Claims**

1. Electrochemical analyzer device, **characterized in that** it comprises the following elements;

   - At least one microcontroller (13) containing an internal analog-digital converter (ADC) and a digital-analog converter (DAC), configured to communicate with other elements in the circuit, and configured to communicate with other computers via at least one internal USB controller (12),
   - At least one control circuit comprising a potentiostat ADC, a potentiostatic signal generator, a USB (12) and a processor, integrated into a single chip,
   - A direct digital synthesizer (DDS) (1) configured to generate a fixed-amplitude sinusoidal signal,
   - At least one reference electrode (RE) (6), at least one counter electrode (CE) (5), at least one working electrode (WE) (7), for performing electrochemical measurements,
   - At least one analog switch (4), for shorting the reference electrode (RE) (6) and the counter electrode (CE) (5),
   - At least one voltage amplifier circuit for potentiostatic measurements, capable of operating within a range of ± 10 V,
   - At least one transimpedance amplifier (TIA) (8) circuit, which allows the current gain to be determined with a digital potentiometer,
   - At least one multiplying DAC (MDAC) circuit, configured to determine the signal amplitude and scale the sinusoidal signal based on feedback from the reference electrode (RE) (6) during impedance analysis,
   - At least one automatic calibration circuit consisting of a digital potentiometer connected in parallel with the Transimpedance Amplifier (TIA) (8) circuit, configured to provide instant calibration to calibrate errors caused by non-ideal situations such as leakage current, voltage differences and temperature,
   - At least one reference voltage generator circuit connected to the power pins of these circuits configured to supply the analog circuits and the working electrode,
   - A background removal circuit (BRC) (9) to eliminate background signals,
   - A low-speed analog-to-digital converter (LSADC) (10) circuit with a resolution ranging from 6-bit to 32-bit and a sampling rate of less than one million per second to read low-frequency signals,
   - A high-speed analog-to-digital converter (HSADC) (11) circuit with a resolution from 6-bit to 32-bit and a sampling rate of more than one million per second to read electrochemical impedance spectroscopy signals,
   - A USB (12) circuit configured to communicate with devices such as computers, tablets, smartphones with the internal USB (12) controller of the microcontroller (13) and configured to receive power from these devices,
   - An operational amplifier (15) in non-inverting feedback configuration, configured to amplify Faradaic currents in potentiostat protocols,
   - An R1 gain resistance (18) and an R2 gain resistance (17), configured to provide voltage from the internal digital-to-analog converter of the microcontroller (13) to the positive pin of the amplifier in the background removal circuit (BRC) (9) and increase the difference $1 + \frac{R2}{R1}$ between this voltage and the output of the Transimpedance amplifier (TIA) (8),

2. A method of operation of an electrochemical analyzer device, **characterized in that** it comprises the process steps of;

• Generating a fixed amplitude sine signal directly by the digital synthesizer (1) (DDS),
• Applying this fixed amplitude signal as a reference signal to the multiplying digital-to-analog converter (2) (MDAC),
• Creating an AC signal with an amplitude ranging from 0.1 mV to 1 V by scaling the reference signal by 6-bit or 24-bit of the multiplying digital-to-analog converter (2) (MDAC),
• Adding the DC voltage to be applied to the electrodes using the internal digital-analogue converter (DAC) of the microcontroller (13) (MC) with the AC signal,
• If a 2 electrode system is used, the analog switch (4) short circuits the reference electrode (RE) and the counter electrode (CE),
• Applying the total result to the CE (5) to keep the voltage applied to the RE at the desired value,
• The current from the sensor flowing through the working electrode (WE) (7) and being converted into voltage through the transimpedance amplifier (8) (TIA),
• The background removal circuit (BRC) (9) increasing the voltage at the TIA output by proportioning it to a voltage proportional to the background voltage to remove non-Faradaic currents,
• Re-amplification of the signal (19) from the TIA with an operational amplifier (15) at a rate determined by R1 gain resistance (18) and R2 gain resistance (17), being the signal (13) base signal provided from the internal DAC of the MC,
• Connecting the output of the operational amplifier to the analog-digital converters (ADC) as input,
• Transmitting the result of the analysis to electronic environments,
• Converting the result to a measurable numerical value.

3. A working method according to claim 2, **characterized in that** it is applied to multiple electrode systems with common reference and common counter electrodes.

4. An analyzer device according to claim 1, **characterized in that** the voltage applied to the said electrodes is adjusted by a multiplying digital-to-analog converter.

**Patentansprüche**

1. Elektrochemische Vorrichtung, **dadurch gekenn-**

zeichnet, dass sie die folgenden Elemente umfasst:

- Mindestens einen Mikrocontroller (13), der einen internen Analog-Digital-Wandler (ADC) und einen Digital-Analog-Wandler (DAC) enthält und für die Kommunikation mit anderen Elementen in der Schaltung und für die Kommunikation mit anderen Computern über mindestens einen internen USB-Controller (12) konfiguriert ist;
- Mindestens einen Steuerkreis, umfassend einen Potentiostat-ADC, einen potentiostatischen Signalgenerator, einen USB (12) und einen Prozessor, integriert in einem einzigen Chip,
- Einen direkten digitalen Synthesizer (DDS) (1), der so konfiguriert ist, dass er ein Sinussignal mit fester Amplitude erzeugt,
- Mindestens eine Referenzelektrode (RE) (6), mindestens eine Gegenelektrode (CE) (5), mindestens eine Arbeitselektrode (WE) (7) zur Durchführung elektrochemischer Messungen,
- Mindestens einen analogen Schalter (4) zum Kurzschließen der Referenzelektrode (RE) (6) und der Gegenelektrode (CE) (5),
- Mindestens eine Spannungsverstärkerschaltung für potentiostatische Messungen, die in einem Bereich von $\pm 10$ V betrieben werden kann,
- Mindestens eine Transimpedanzverstärker- (TIA-) (8) Schaltung, die es ermöglicht, die Stromverstärkung mit einem digitalen Potentiometer zu bestimmen,
- Mindestens eine Multiplikations-DAC- (MDAC-)Schaltung, die so konfiguriert ist, dass sie die Signalamplitude bestimmt und das Sinussignal basierend auf der Rückmeldung von der Referenzelektrode (RE) (6) während der Impedanzanalyse skaliert,
- Mindestens eine automatische Kalibrierschaltung, bestehend aus einem digitalen Potentiometer, das parallel zur Transimpedanzverstärker- (TIA-) (8) Schaltung geschaltet ist und so konfiguriert ist, dass es eine sofortige Kalibrierung zur Korrektur von Fehlern ermöglicht, die durch nicht ideale Situationen wie Leckstrom, Spannungsunterschiede und Temperatur verursacht werden,
- Mindestens eine Referenzspannungsgeneratorschaltung, die mit den Stromanschlüssen dieser Schaltungen verbunden ist, die so konfiguriert sind, dass sie die analogen Schaltungen und die Arbeitselektrode versorgen,
- Eine Hintergrundentfernungsschaltung (BRC) (9) zum Eliminieren von Hintergrundsignalen,
- Eine Schaltung mit einem Analog-Digital-Wandler mit niedriger Geschwindigkeit (LSADC) (10) mit einer Auflösung im Bereich von 6 Bit bis 32 Bit und einer Abtastrate von weniger als einer Million pro Sekunde zum Lesen von Niederfrequenzsignalen,

- Eine Schaltung mit einem Analog-Digital-Wandler mit hoher Geschwindigkeit (HSADC) (11) mit einer Auflösung von 6 Bit bis 32 Bit und einer Abtastrate von mehr als einer Million pro Sekunde zum Lesen von elektrochemischen Impedanzspektroskopiesignalen,

- Eine USB- (12) Schaltung, die so konfiguriert ist, dass sie mit Vorrichtungen wie Computern, Tablets und Smartphones über die interne USB-(12) Steuerung des Mikrocontrollers (13) kommuniziert und von diesen Vorrichtungen mit Strom versorgt wird,

- Einen Operationsverstärker (15) in nicht invertierender Rückkopplungskonfiguration, der so konfiguriert ist, dass er Faraday-Ströme in Potentiostat-Protokollen verstärkt,

- Einen Verstärkungswiderstand R1 (18) und einen Verstärkungswiderstand R2 (17), die so konfiguriert sind, dass sie eine Spannung vom internen Digital-AnalogWandler des Mikrocontrollers (13) an den positiven Pin des Verstärkers in der Hintergrundentfernungsschaltung (BRC) (9) liefern und die Differenz $1 + \frac{R2}{R1}$ zwischen dieser Spannung und dem Ausgang des Transimpedanzverstärkers (TIA) (8) zu erhöhen,

2. Verfahren zum Betrieb einer elektrochemischen Analysevorrichtung, **dadurch gekennzeichnet, dass** es die folgenden Prozesse umfasst:

- Erzeugen eines Sinussignals mit fester Amplitude direkt durch den digitalen Synthesizer (1) (DDS),
- Anwenden dieses Signals mit fester Amplitude als Referenzsignal auf den multiplizierenden Digital-AnalogWandler (2) (MDAC),
- Erstellen eines Wechselstromsignals mit einer Amplitude im Bereich von 0,1 mV bis 1 V durch Skalierung des Referenzsignals mit 6 Bit oder 24 Bit des multiplizierenden Digital-Analog-Wandlers (2) (MDAC),
- Hinzufügen der an die Elektroden anzuwendenden Gleichspannung durch Verwendung des internen Digital-Analog-Wandlers (DAC) des Mikrocontrollers (13) (MC) mit dem Wechselstromsignal,
- Wenn ein 2-Elektroden-System verwendet wird, schließt der Analogschalter (4) die Referenzelektrode (RE) und die Gegenelektrode (CE) kurz,
- Anwenden des Gesamtergebnisses auf die CE (5), um die an den RE angelegte Spannung auf dem gewünschten Wert zu halten,
- Der Strom vom Sensor fließt durch die Arbeitselektrode (WE) (7) und wird durch den Transimpedanzverstärker (8) (TIA) in Spannung umgewandelt,
- Die Hintergrundentfernungsschaltung (BRC) (9) erhöht die Spannung am TIA-Ausgang, indem sie diese proportional zu einer Spannung anpasst, die proportional zur Hintergrundspannung ist, um nicht-faradaysche Ströme zu entfernen,
- Neuverstärkung des Signals (19) vom TIA mit einem Operationsverstärker (15) mit einer Rate, die durch den Verstärkungswiderstand R1 (18) und den Verstärkungswiderstand R2 (17) bestimmt wird, wobei es sich bei dem Signal (13) um das Basissignal handelt, das vom internen DAC des MC bereitgestellt wird,
- Verbinden des Ausgangs des Operationsverstärkers mit den Analog-Digital-Wandlern (ADC) als Eingang,
- Übertragen der Analyseergebnisse in elektronische Umgebungen,
- Umwandeln des Ergebnisses in einen messbaren numerischen Wert.

3. Arbeitsverfahren nach Anspruch 2, **dadurch gekennzeichnet, dass** es auf Mehrfachelektrodensysteme mit gemeinsamen Referenz- und Gegenelektroden angewendet wird.

4. Analysevorrichtung nach Anspruch 1, **dadurch gekennzeichnet, dass** die an die Elektroden angelegte Spannung durch einen multiplizierenden Digital-AnalogWandler eingestellt wird.

**Revendications**

1. Dispositif d'analyse électrochimique, **caractérisé en ce qu'**il comprend les éléments suivants ;

- au moins un micro dispositif de commande (13) contenant un convertisseur analogique-numérique (ADC) interne et un convertisseur numérique-analogique (DAC), configuré pour communiquer avec d'autres éléments du circuit et configuré pour communiquer avec d'autres ordinateurs par le biais d'au moins un dispositif de commande USB interne (12),
- au moins un circuit de commande comprenant un convertisseur analogique-numérique potentiostatique, un générateur de signal potentiostatique, un port USB (12) et un processeur, intégrés dans une seule puce,
- un synthétiseur numérique direct (DDS) (1) configuré pour générer un signal sinusoïdal d'amplitude fixe,
- au moins une électrode de référence (RE) (6), au moins une contre-électrode (CE) (5), au moins une électrode de travail (WE) (7), pour réaliser des mesures électrochimiques,

- au moins un commutateur analogique (4), pour court-circuiter l'électrode de référence (RE) (6) et la contre-électrode (CE) (5),

- au moins un circuit amplificateur de tension pour mesures potentiostatiques, capable de fonctionner dans une plage de $\pm 10$ V,

- au moins un circuit amplificateur de transimpédance (TIA) (8), qui permet de déterminer le gain en courant à l'aide d'un potentiomètre numérique,

- au moins un circuit DAC multiplicateur (MDAC), configuré pour déterminer l'amplitude du signal et mettre à l'échelle le signal sinusoïdal sur la base de la rétroaction de l'électrode de référence (RE) (6) pendant l'analyse d'impédance,

- au moins un circuit d'étalonnage automatique composé d'un potentiomètre numérique connecté en parallèle avec le circuit amplificateur de transimpédance (TIA) (8), configuré pour fournir un étalonnage instantané afin de corriger les erreurs causées par des situations non idéales telles que le courant de fuite, les différences de tension et la température,

- au moins un circuit générateur de tension de référence connecté aux broches d'alimentation de ces circuits configurés pour alimenter les circuits analogiques et l'électrode de travail,

- un circuit de suppression de fond (BRC) (9) pour éliminer les signaux de fond,

- un circuit convertisseur analogique-numérique à basse vitesse (LSADC) (10) avec une résolution allant de 6 bits à 32 bits et une fréquence d'échantillonnage inférieure à un million par seconde pour lire les signaux basse fréquence,

- un circuit convertisseur analogique-numérique à haute vitesse (HSADC) (11) avec une résolution de 6 bits à 32 bits et une fréquence d'échantillonnage supérieure à un million par seconde pour lire les signaux de spectroscopie d'impédance électrochimique,

- un circuit USB (12) configuré pour communiquer avec des dispositifs tels que des ordinateurs, des tablettes, des smartphones avec le dispositif de commande USB (12) interne du micro dispositif de commande (13) et configuré pour recevoir l'alimentation de ces dispositifs,

- un amplificateur opérationnel (15) en configuration de rétroaction non inverseuse, configuré pour amplifier les courants faradiques dans les protocoles potentiostatiques,

- une résistance de gain R1 (18) et une résistance de gain R2 (17), configurées pour fournir une tension du convertisseur numérique-analogique interne du micro dispositif de commande (13) à la broche positive de l'amplificateur dans le circuit de suppression de fond (BRC) (9) et augmenter la différence $1 + \frac{R2}{R1}$ entre cette tension et la sortie de l'amplificateur de transimpédance (TIA) (8).

2. Procédé de fonctionnement d'un dispositif d'analyse électrochimique, **caractérisé en ce qu'**il comprend les étapes de traitement suivantes :

    • la génération d'un signal sinusoïdal d'amplitude fixe directement par le synthétiseur numérique (1) (DDS),
    • l'application de ce signal d'amplitude fixe comme signal de référence au convertisseur numérique-analogique multiplicateur (2) (MDAC),
    • la création d'un signal CA avec une amplitude allant de 0,1 mV à 1 V en mettant à l'échelle le signal de référence par 6 bits ou 24 bits du convertisseur numérique-analogique multiplicateur (2) (MDAC),
    • l'ajout de la tension continue à appliquer aux électrodes à l'aide du convertisseur numérique-analogique (DAC) interne du micro dispositif de commande (13) (MC) avec le signal CA,
    • si un système à 2 électrodes est utilisé, le commutateur analogique (4) court-circuite l'électrode de référence (RE) et la contre-électrode (CE),
    • l'application du résultat total à la CE (5) pour maintenir la tension appliquée à la RE à la valeur souhaitée,
    • le courant provenant du capteur circulant à travers l'électrode de travail (WE) (7) et étant converti en tension à travers l'amplificateur de transimpédance (8) (TIA),
    • le circuit de suppression de fond (BRC) (9) augmentant la tension à la sortie du TIA en la proportionnant à une tension proportionnelle à la tension de fond pour supprimer les courants non faradiques,
    • la réamplification du signal (19) provenant du TIA avec un amplificateur opérationnel (15) à une fréquence déterminée par la résistance de gain R1 (18) et la résistance de gain R2 (17), le signal (13) étant le signal de base fourni par le DAC interne du MC,
    • la connexion de la sortie de l'amplificateur opérationnel aux convertisseurs analogique-numérique (ADC) en tant qu'entrée,
    • la transmission du résultat de l'analyse aux environnements électroniques,
    • la conversion du résultat en une valeur numérique mesurable.

3. Procédé de travail selon la revendication 2, **caractérisé en ce qu'**il est appliqué à des systèmes à électrodes multiples avec des électrodes de référence et des contre-électrodes communes.

4. Dispositif d'analyse selon la revendication 1, **caractérisé en ce que** la tension appliquée auxdites électrodes est ajustée par un convertisseur numérique-analogique multiplicateur.

$\frac{A\sin(wt)}{2^n}$

$A\sin(wt)$

FIGURE 1

FIGURE 2

**FIGURE 3**

**FIGURE 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018224394 A1

### Non-patent literature cited in the description

- **CARMINATI, M.** ; **FERRARI, G** ; **SAMPLIETRO, M.** Attofarad Resolution Potentiostat for Electrochemical Measurements on Nanoscale Biomolecular Interfacial Systems. *Review of Scientific Instruments*, 2009, vol. 80, 124701

- **MATSUBARA, Y.** A Small yet Complete Framework for a Potentiostat, Galvanostat, and Electrochemical Impedance Spectrometer. *J. Chem. Educ.*, 2021, vol. 98, 3362-3370